(19) Europäisches Patentamt

European Patent Office

Office européen des brevets

(11) **EP 3 941 400 B1**

(12) **EUROPEAN PATENT SPECIFICATION**

(45) Date of publication and mention
of the grant of the patent:
**26.04.2023 Bulletin 2023/17**

(21) Application number: **20717244.6**

(22) Date of filing: **20.03.2020**

(51) International Patent Classification (IPC):
***A61F 11/08*** *(2006.01)*

(52) Cooperative Patent Classification (CPC):
**A61F 11/08; A61F 11/085**

(86) International application number:
**PCT/GB2020/050755**

(87) International publication number:
**WO 2020/188296 (24.09.2020 Gazette 2020/39)**

(54) **EAR PROTECTION**

GEHÖRSCHUTZ

PROTECTEUR D'OREILLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.03.2019 GB 201903920**

(43) Date of publication of application:
**26.01.2022 Bulletin 2022/04**

(73) Proprietor: **Minuendo AS
0153 Oslo (NO)**

(72) Inventors:
• **KVALØY, Olav
0153 Oslo (NO)**
• **AURVIK, Håkon
0153 Oslo (NO)**
• **PETTERSEN, Odd Kr. Ø.
0153 Oslo (NO)**
• **SWENDGAARD, Erik
0153 Oslo (NO)**

(74) Representative: **Dehns
St. Bride's House
10 Salisbury Square
London EC4Y 8JD (GB)**

(56) References cited:
**WO-A1-2013/080005     WO-A1-2017/203218
WO-A1-2018/070876     US-B2- 7 478 702
US-B2- 7 512 243     US-B2- 8 161 975**

**Description**

The present invention relates to ear protection used, for example, to reduce the intensity of sounds experienced by a user.

Exposure to high intensity noises can cause damage to a person's hearing. The damaging effects are increased when a person is frequently exposed to loud noises. In extreme cases, frequent exposure to loud noises can cause noise-induced hearing loss. Therefore, in order to protect hearing it is necessary to reduce the effects of continuous, intermittent and impact noises. As a result of the increasing awareness of the damaging effects of loud noises, for example from industrial sources, there are now various industry requirements for personnel to use ear protection. There are many situations in which personnel may be exposed to loud noises, for example when operating loud machinery. A common form of noise protection widely used are earplugs, these reduce the intensity of the sound entering a person's ears and thus reduce the damaging effects of high intensity noises.

There are two main types of earplugs that are commonly used: passive earplugs and active earplugs. Passive earplugs attenuate the intensity of all levels of sound equally, i.e. they provide a uniform level of attenuation, regardless of the intensity of sound present, for example, a reduction of 20 dB. Passive earplugs come in various forms including: foam, silicon, flanged and custom moulded earplugs. Passive earplugs are typically inserted into a user's ear canal. Passive earplugs use the material of the earplug itself to attenuate the sound which passes through it. As the incident sound passes through the earplug, the sound is attenuated by the material of the earplug. Some sound will propagate through the earplug and pass out of the earplug into the air volume of the user's ear canal where it will be detected by the user. The intensity of the sound will be reduced and thus the risk of damage to the user's hearing may be reduced. Unfortunately, however, such passive earplugs also change other properties of the sound as the attenuation is usually frequency dependent and as a result the sound quality is often reduced. This may be problematic, for example for musicians as they need to hear sounds in high fidelity to ensure that what they hear is actually what is being played.

In addition, if they are inserted properly, the fixed level of attenuation provided by passive earplugs is relatively high. As a result, users of passive earplugs typically have to periodically remove them in order to be communicate orally with fellow workers. The inconvenience associated with having to repeatedly remove and replace the earplugs, depending on the noise levels, may lead to reduced compliance with requirements to wear the earplugs in certain situations.

A device according to the preamble of claim 1 is known from the document US-B-7478702.

The aforementioned disadvantages can be overcome using active earplugs which comprise electronic components which enable the earplug to attenuate high intensity sounds whilst being relatively acoustically transparent and providing only low levels of attenuation when the surrounding sound has low intensity. Active earplugs often comprise a passive earplug used in conjunction with a microphone on the external side of the earplug and a speaker on the internal side of the earplug. Active earplugs typically listen to the sound on the outside of the earplug, and then replay it to a user via the speaker at a reduced intensity. Active earplugs can employ control circuitry to apply different levels of attenuation at different times or different frequencies - known as 'adaptive attenuation'. Some other systems contain circuitry that detects the incident sound and produces an out-of-phase signal that destructively interferes with the incident sound thus reducing the intensity of the incident sound as it propagates into the user's ear canal - this is often known as 'active noise cancelling'. One of the disadvantages of active earplugs is that they are often relatively expensive due to their electrical components. Additionally, active earplugs often have a relatively high power consumption due to the need to constantly monitor and replay detected sound. The present invention seeks to address or mitigate the problems outlined above and according to a first aspect there is provided a device, for insertion into an ear canal of a mammalian subject, comprising:

a body, having at least one sound path extending therethrough;:

a first adjustable acousto-mechanical portion comprising an adjustable channel forming at least part of the sound path;
a second adjustable acousto-mechanical portion, arranged acoustically in series with the first adjustable acousto-mechanical portion, comprising an adjustable membrane; and
an adjustment arrangement for simultaneously adjusting the first and second adjustable acousto-mechanical portions to alter an acoustic response of the at least one sound path.

The Applicant has recognised that with the claimed arrangement of the adjustable channel and the adjustable membrane, it is possible to achieve an acoustic response of the sound path which does not significantly reduce the quality of the sound passing through the sound path whilst maintaining the ability to control the sound, e.g. by attenuating the sound. This is because it allows the changes in the channel and the membrane as they are adjusted to complement one another in maintaining a favourable acoustic response.

As will be understood by those skilled in the art, the acoustic response of the sound path should be understood to be how the sound path affects the sound which passes through it. The acoustic response of the sound path may change the frequency, amplitude and/or phase of the sound passing through it and thus ultimately change the sound heard by a user of the device.

As will be appreciated by those skilled in the art, devices in accordance with the invention advantageously do not necessarily require the presence of the complex electronic circuitry included in active earplugs, such as a speaker to replay sounds to a user, whilst maintaining the ability to control the sound and so avoid the need to remove and replace earplugs to adapt to a changing sound environment. As a result, the device may have a zero or very low power consumption when compared to typical active earplugs. Additionally, when compared to purely passive earplugs, devices in accordance with the invention advantageously may maintain the quality of the sound passing through the device.

The adjustment arrangement may comprise any suitable arrangement for adjusting the first adjustable acousto-mechanical portion and the second adjustable acousto-mechanical portion. In a set of embodiments the adjustment arrangement comprises a first actuator for adjusting the first adjustable acousto-mechanical portion and a second actuator for adjusting the second adjustable acousto-mechanical portion. The first and second actuators may, for example, be connected to a single controller capable of simultaneously controlling each of the first and second adjustable acousto-mechanical portions. Such an arrangement may be particularly advantageous when the first and second adjustable acousto-mechanial portions require adjustment by differing amounts, e.g. due to the need to achieve a particular acoustic response. Having separate actuators for each adjustable acousto-mechanical portion may make it possible to adjust one of the portions through a greater proportion of its physical range of movement than the other if required in a particular instance to achieve a desired response.

In an alternative set of embodiments, the adjustment arrangement comprises a common actuator arranged to adjust both the first and second acousto-mechanical portions simultaneously. The Applicant has found that the use of such a common actuator may be advantageous, for example in embodiments wherein the device is electrically powered as it may reduce the amount of power required to adjust the first and second adjustable acousto-mechanical portions. It may also simplify the construction of the device. Of course by suitable design of such an actuator, e.g. to include one or more levers or members of differing stiffness, different amounts of movement may be imparted to the respective acousto-mechanical portions for a given input movement.

In a set of embodiments the or each actuator comprises an electric motor. The use of an electronic motor may advantageously mean that the device can automatically adjust the acousto-mechanical portions without requiring physical input from a user. For example the use of an electric motor may mean that the device can automatically adjust to attenuate the sound in the presence of a loud environment, without requiring the user to take any action, thereby helping to ensure that the sound path has an appropriate acoustic response for the acoustic environment the user is in, thus protecting the user.

In another set of embodiments, the or each actuator comprises a user operable member arranged to operate at least part of the adjustment arrangement. The user operable member may directly drive the adjustment arrangement. The user operable member may, for example, comprise a rotatable knob arranged to adjust the first and second adjustable acousto-mechanical portions. The Applicant has recognised that a user operable member arranged to operate part of the adjustment arrangement may advantageously simplify the device and potentially reduce its cost. Through the use of a user operable member, it may be possible to achieve a device which does not comprise any electrical/electronic components, thereby potentially providing a device which does not require electrical power. Achieving a device which does not require power may mean that the device is more frequently used as users do not have to concern themselves with ensuring that the device has enough battery power for operation. This may help to improve compliance with, for example, industry requirements to use hearing protection.

The first adjustable acousto-mechanical portion comprising the adjustable channel may be adjusted in any appropriate manner in order to achieve the desired acoustic response. In a set of embodiments the adjustment arrangement is configured to adjust a length of the adjustable channel. The Applicant has recognised that adjusting the length of the adjustable channel may in general increase the effect of the channel. Additionally or alternatively, the adjustment arrangement is configured to adjust a width of the adjustable channel. The Applicant has found that adjusting the width of the adjustable channel may serve to adjust the specific acoustic properties of the sound path. For example, decreasing the width of the channel will typically increase the effective acoustic mass and the acoustic loss of the channel and vice versa. Conversely decreasing the length of the channel will typically decrease the effective acoustic mass and the acoustic loss of the channel and vice versa. In other words the acoustic mass and loss typically have a positive relationship with the length of the channel and a negative relationship with the width of the channel. The terms acoustic mass and acoustic loss are well known to those skilled in the art but will be further explained later.

As will be appreciated, the length and width of the adjustable channel may be adjusted independently of one another, or simultaneously together. In a potentially overlapping set of embodiments, the adjustment arrangement is configured to adjust a shape of the adjustable channel.

The adjustable channel may be defined by any suitable structure within the device. For example, the channel may simply comprise a cylindrical or other shaped channel extending through the body of the device. Adjustment of such a channel may, for example, comprise constricting and expanding the body so as to decrease/increase the size of the channel, or comprise providing a constriction at the entrance to or exit from the channel or part way along the channel. In a set of embodiments, the channel comprises an adjustable barrier member. In a further set of embodiments, however, the channel is defined by a space between a wall of a cavity within the body and a piston arranged in the cavity, wherein adjustment of the channel is achieved by moving the piston relative to the cavity. The cross-section of the channel will depend on the shape of the wall of the cavity and the outer profile of the piston. The piston may have a complementary sectional shape to the wall of the cavity, e.g. if the wall has a circular sectional shape, the piston may have a circular sectional shape. In such an example, the channel defined between the wall and piston would effectively be an elongate annular channel. The Applicant has recognised that the arrangement of a piston in the cavity provides for a relative simple means to adjust the length and/or cross-sectional area and/or shape of the channel.

The piston may be arranged in the device in any suitable manner such that it can be moved relative to the cavity. For example, the piston may be a part of, or attached to, a linear actuator capable of moving the piston into, and out of, the cavity. In a set of embodiments, the piston is arranged to move axially within the cavity and the device comprises at least one resilient member arranged to bias the piston out of the cavity, wherein the adjustment arrangement comprises an actuation member arranged to drive the piston against the resilient bias axially into the cavity. The actuation member may be driven by an electric motor or a user operable member. The Applicant has recognised that the provision of a resilient member arranged in the manner according to the above set of embodiments means that the actuation member needs only to be able to drive movement in one axial direction as the resilient member is arranged to drive movement of the piston in the other axial direction. This may simplify the manufacture and construction of the device. In a set of embodiments, the actuation member is arranged to rotate relative to the piston, and the device further comprises an arrangement for converting rotational movement of the actuation member into axial movement of the piston. The resilient member may be integrally provided with the piston. In a further set of embodiments, a plurality of resilient members is provided. In another set of embodiments, the at least one resilient member is in the form of a resilient arm extending between the piston and the body.

In another set of embodiments, the piston comprises a threaded portion, arranged to engage with a threaded portion on the body such that rotation of the piston causes linear movement of the piston within the cavity. Such a set of embodiments may utilise a motor to drive movement of the piston, or a user operable member. As will be appreciated by those skilled in the art, the pitch of the threaded portion may be chosen to allow for highly controlled movement of the piston within the cavity. Such control may be required in order to precisely control the acoustic response of the adjustable channel. The threaded engagement between the piston and the body, specifically the static frictional force which arises between the threaded portions, may also mean that the piston is held in its position without requiring action from a further component e.g. a motor. This may mean that once the piston has been moved to its desired position any power being supplied, for example, to a motor, may be turned off. This may help to reduce the power consumption of the device.

Of course, in addition or alternatively, the piston may be arranged to move in other directions other than just axially. For example, the piston may be moved at a non-zero angle to the axis, be translated from side-to-side within the cavity or even twisted, in order to adjust the channel so as to achieve a desired acoustic response.

In a set of embodiments, the piston is arranged such that it can be held stable in a plurality of different positions in the cavity. This may be achieved due to the presence of, for example, static friction as described above with respect to the embodiment wherein the piston comprises a threaded portion. Alternatively, the device may comprise different means for holding the piston stable. For example, the piston and body may each comprise a series of recesses and corresponding protrusions acting therebetween to hold the piston stable when the recesses and protrusions are in engagement with one another.

The Applicant has recognised that in order to appropriately control the acoustic response of the sound path, it may be necessary to adjust the length and cross-section of the adjustable channel simultaneously. In a further set of embodiments, the cavity and the piston each have a frusto-conical shape such that the adjustable channel has the form of a frusto-conical shell. The Applicant has recognised that in such an arrangement axial movement of the piston within the cavity may simultaneously adjust both the length of the channel and the width. It may also mean that a relatively large axial movement can be converted into a relatively small change in width. This may help to simplify the manufacture and construction of the device and to provide fine control over the width The piston and cavity are preferably shaped so that the channel remains of uniform shape throughout the travel of the piston but this is not essential.

As discussed previously, a common actuator may be provided to adjust both the adjustable channel and the adjustable membrane simultaneously. Such a common actuation may be achieved in any number of ways. In embodiments comprising a piston, in a further set of embodiments the piston is arranged such that movement of the piston adjusts a tension of the membrane. The piston may be coupled to the membrane in order to adjust the tension of the membrane in any suitable manner. For example, the piston may extend through the cavity and be directly coupled to the membrane, e.g. it may physically come in direct contact with the membrane in order to deform it. In a set of embodiments, however, the piston is coupled to a resilient member arranged to act on the membrane, or a part of the body to which the membrane is attached. As will be appreciate by those skilled in the art, the arrangement of a resilient member acting in-between the piston and the membrane may scale down the movement of the piston and its effect on the membrane. For example, the resilient member may convert millimetre movement of the piston into micrometre movement with respect to the membrane. Depending on the piston and the membrane, this may be necessary to achieve the desired acoustic response of the channel. The resilient member may be integrally formed as part of the body or the piston, or alternatively it may be a separate, independent component.

The resilient member may be provided by any suitable means. In a set of embodiments, however, the resilient member comprises a central portion, coupled to the piston, and a plurality of legs extending from the central portion arranged to act on the membrane, or a part of the body to which the membrane is attached. The Applicant has recognised that such a resilient member comprising a plurality of legs may more evenly distribute the force applied to the membrane which may result in a more uniform adjustment, e.g. tensioning, of the membrane.

In embodiments in which the piston physically comes into contact with the membrane in order to deform and therefore adjust the tension in the membrane, the Applicant has noticed that the level of attenuation provided by the device increases substantially when the piston first contacts the membrane compared to the level of attenuation provided when the piston is not in contact with the membrane and would increase rapidly thereafter, even with small movements. The Applicant has recognised that this effect is undesirable but that it may be reduced in some embodiments by including a compressible portion in the piston. Therefore, in a set of embodiments, the piston comprises a compressible portion.

When the adjustable member contacts the membrane, the compressible portion of the adjustable member is compressed (and/or deformed), reducing the force exerted on the membrane by the adjustable member and resulting in a smaller increase in the tension of the membrane. Therefore, the level of attenuation provided by the device may increase more smoothly and more gradually when the piston contacts the membrane compared with a rigid piston (i.e. with no compressible portion), to allow finer control over the attenuation provided by the device.

The compressible portion could be provided by any part of the piston, but in a set of embodiments the compressible portion of the piston contacts the surface of the membrane. This may aid the stability of the piston.

The piston may be arranged to have any suitable and desirable shape. The piston may comprise a portion positioned within the channel and another portion extending therefrom to contact the membrane. There may be embodiments in which the compressible portion (which comprises part of the piston) does not have the same shape as the remaining portion(s) of the piston. For example, the compressible portion may be a cylinder with a smaller diameter than the remaining portion(s) of the piston.

The compressible portion of the piston may be shaped to be compressible (e.g. comprising a spring) and/or the compressible portion may be formed from a layer of inherently compressible material. Preferably, the compressible portion of the piston is formed from an elastically compressible material. Whilst the Applicant has appreciated that the piston could be formed from any suitable and desirable compressible material, in a set of embodiments, the deformable portion is formed from thermoplastic elastomers or foam materials, e.g. polyurethane foam.

In a set of embodiments the piston, or the rest of the piston apart from the compressible portion, is formed from a rigid material. Whilst the Applicant has appreciated that the piston could be formed from any suitable and desirable material, preferably the piston is formed from a plastic. For example, the piston is formed from a rigid plastic e.g. polycaprolactam (PA6), acrylonitrile butadiene styrene (ABS) or polyoxymethylene (POM).

In a set of embodiments, the piston comprises a central axis which is perpendicular to a plane or at least a central tangent plane of the membrane. In a set of embodiments, the piston is rotationally symmetric about a central axis. This may help to ensure a uniform force is exerted on the membrane when the piston is in contact with the membrane.

The piston may be in contact with membrane throughout its travel. However, in a set of embodiments the piston has a position wherein the piston is not in contact with the membrane. In this position, the piston does not exert a force on the membrane and therefore, the piston does not alter the tension of the membrane from a base value.

It will be appreciated by the skilled person that when the piston is in the non-contact position, the base level of attenuation provided by the device is lower than the level of attenuation provided by the device when the piston is in contact with the membrane. This is because when the piston is in contact with the membrane, the tension in the membrane is increased and therefore the attenuation of sounds by the membrane increases. The measures provided in accordance with the invention however mean that there is much less of a sharp change in performance between non-contact and contact as discussed previously than there would otherwise have been.

In a set of embodiments, the piston has a plurality of positions wherein the piston is in contact with the membrane. The plurality of positions of the piston correspond to a plurality of magnitudes of force applied to the membrane, and therefore a plurality of tensions in the membrane. For example, as the piston is moved further towards the membrane, the piston exerts a greater force on the membrane and therefore the tension of the membrane is increased. The attenuation of the membrane is therefore greater and a higher level of attenuation is provided by the device.

The acoustic response of the at least one sound path may additionally be altered by providing a barrier member.

In a set of embodiments, the barrier member comprises an adjustable closure, such a lid which could be arranged to hinge or slide. Preferably, the barrier member has at least an open position and a closed position. In the open position, sound may be able to propagate through the device as previously described i.e. relatively attenuated by the barrier member. In the closed position, sound may be substantially attenuated by the barrier member.

The barrier member may be configured to provide a plurality or a continuum of different positions between, for example, the open and closed position. The membrane may be in the form of a relatively thin sheet of material arranged on, or in, the device and may have any appropriate shape, for example a circular shape. In an exemplary set of embodiments the membrane is made from a low density plastic film e.g. polyethylene terephthalate. The Applicant has recognised that a circular-shaped membrane may allow for more uniform adjustment of the membrane, e.g. tensioning of the membrane, when compared to membranes having alternative shapes, e.g. square shaped membranes. The membrane may be integral to the body. For example, the membrane may be integrally moulded with the body, or the body may be milled in order to form the membrane. However, the Applicant has recognised that integrally providing the membrane with the body may be complicated to manufacture. In a set of embodiments, the membrane is a separate component attached to the body. In a further set of embodiments the body defines a circumferential rim to which the membrane is attached. This may allow for more simple manufacture of the body. Additionally, it may allow the body and the membrane to be manufactured from different materials which may be necessary in order to provide a sound path having the required acoustic response. In embodiments comprising a resilient member, the resilient member may act on the rim in order to adjust the tension on the membrane. Such an arrangement allows the membrane to be adjusted without directly acting on the membrane.

In a set of embodiments, the membrane comprises at least one corrugation. The Applicant has appreciated that finer control over the attenuation provided by the device can be achieved, particularly in embodiments in which the piston is arranged to contact the membrane, by implementing a tensioned membrane that includes at least one corrugation. When the piston contacts the membrane, the corrugation is typically (at least partially) stretched due to the force the piston exerts on the membrane and a smaller increase in the tension of the membrane may be observed for a given applied force. Therefore, the level of attenuation provided by the device may increase more gradually when the piston contacts the membrane compared to a membrane without any corrugation. This may therefore provide a similar effect to providing the piston with a compressible portion and may be implemented instead of or as well as that feature when the piston contacts the membrane.

In a set of embodiments, the corrugation comprises a ridge. In a potentially overlapping set of embodiments, the corrugation comprises an indentation. Depending on the orientation of the membrane, an identical corrugation could be described as either a ridge or an indentation. Preferably the maximum height of the ridge above or depth of the indentation below the plane of the membrane is in the range of 0.02 mm to 2 mm e.g. 0.1 mm to 1 mm e.g. 0.3 mm.

In a set of embodiments, the corrugation is arranged on the membrane such that the piston does not contact the corrugation. The piston may deform the corrugation by applying a force to another part of the membrane which is transmitted to the corrugation.

In a set of embodiments, the piston is arranged to contact the membrane in the geometric centre of the membrane. In such embodiments and when the membrane is circular, the corrugation may be arranged on the membrane at a distance from the geometric centre of the membrane in the range of 30% to 90% of the radius of the membrane.

In a set of embodiments, the corrugation is circular and centred on the geometric centre of the membrane. The ridge or indentation preferably has a uniform cross-section (e.g. the circular corrugation has a constant cross-section throughout the circumference of the circle formed), such that the corrugation is deformed uniformly by the piston. This may help to ensure that the quality of the sound is not significantly reduced by the device.

In a set of embodiments, the membrane comprises a plurality of corrugations. Different corrugations, or different subsets of the corrugations within the plurality of corrugations, may have different shapes and sizes. However, preferably the plurality of corrugations all have the same shape. Corrugation which are uniform in shape may deform similarly under the same force. Therefore, implementing a plurality of uniform corrugations (especially in combination with a uniform arrangement of corrugations on the adjustable membrane) may help the level attenuation of the device to be varied smoothly and predictably.

The Applicant has envisaged a particular arrangement in which the plurality of corrugations comprises a subset of circular ridges and subset of circular indentations, which may be centred on the geometric centre of the membrane. The various circular indentations and ridges would have various diameters, and therefore could be arranged at various distances (i.e. locations) from the geometric centre of the adjustable membrane. The circular indentations and ridges may be arranged to alternate, e.g. if the innermost circular corrugation is an indentation, the second innermost circular corrugation is a ridge, the third innermost circular corrugation is an indentation etc.

In embodiments comprising a user operable member to operate at least part of the adjustment arrangement, when a user hears, or expects to hear, a particular type of sound, they may operate the user operable member to adjust the adjustment arrangement and thus alter the acoustic response of the at least one sound path. Such a set of embodiments may be suitable for achieving a relatively inexpensive device. In a set of embodiments, however, the device further comprises a controller arranged to control the adjustment arrangement so as to alter the acoustic response of the at least one sound path. The inclusion of a controller may allow the device to control the adjustment arrangement in a more sophisticated manner in order to provide the most appropriate acoustic response of the sound path. It may, for example, try to achieve the highest sound quality whilst providing the necessary amount of attenuation. The controller may comprise a series of other components connected thereto or integrally provided therewith, for example a microphone or a transceiver. Such a set of embodiments may also advantageously automatically adjust the acoustic response of the sound path based on a detected sound environment in which the device is present. Additionally, or alternatively, the device may comprise a user input, e.g. in the form of at least one button, which enables the user to control operation of the device. This may, for example, allow a user to select a particular mode of operation.

Some preferred embodiments of the present invention will now be described, by way of example only, and with reference to the accompanying drawings, in which:

Fig. 1 shows an isometric view of a device in accordance with an embodiment of the present invention;
Fig. 2 shows a view of the underside of the device seen in Fig. 1;
Fig. 3 shows an exploded view of the device seen in Fig. 1
Figs. 4a-4b show the actuation member, which forms part of the device in Fig. 1, in isolation;
Fig. 5 shows a piston, which forms part of the device seen in Fig. 1, in isolation;
Fig. 6 shows part of the body of the device seen in Fig. 1 in isolation;
Fig. 7 shows part of the body, of the device seen in Fig. 1, along with the membrane;
Figs. 8a-8b are illustrations demonstrating how the relative position of the piston in the cavity adjusts the channel;
Fig. 9a-9b are illustrations demonstrating how the tension on the membrane is adjusted;
Figs. 10a-10b show a cross-sectional view through the device seen in Fig. 1 in an open configuration;
Figs. 11-11b show perspective views of the device seen in Fig. 1 in a closed configuration;
Figs. 12a-12b show cross-sectional views through the device in the closed configuration;
Fig. 13 shows a perspective view of a device in accordance with a second embodiment of the present invention;
Figs. 14a-14b show cross-sectional views of the device seen in Fig. 13, in an open configuration;
Figs. 15a-15b show cross-sectional views of the device seen in Fig. 13 in a closed configuration;
Fig. 16a shows an isometric view of part of a hearing protection a device in accordance with a third embodiment of the present invention;
Fig. 16b shows an alternative isometric view of the device seen in Figure 16a;
Fig. 17a shows an exploded view of parts of the device seen in Figure 16a;
Fig. 17b shows an isolated isometric view of the body of the device seen in Figure 16a;
Fig. 18 shows the piston, user operable member and the membrane of the device seen in Figure 16a;
Fig. 19 shows an isolated isometric view of the piston and user operable member of the device seen in Figure 16a;
Fig. 20 shows a cross-sectional view through certain components of the device seen in Figure 16a in a first configuration;
Fig. 21 shows a cross-sectional view through certain components of the device seen in Figure 16a in a second configuration;
Fig. 22 shows a cross-sectional view through certain components of the device seen in Figure 16a in a third configuration;
Fig. 23 shows another cross-sectional view through the device seen in Figure 16a in the first configuration;
Fig. 24 shows another cross-sectional view through the device seen in Figure 16a in the second configuration;
Fig. 25 shows another cross-sectional view through the device seen in Figure 16a in the third configuration;
Fig. 26a-26b show cross-sectional views of the device in accordance with a fourth aspect of the present invention; and
Fig. 27 is a graph showing frequency response of an embodiment of the invention compared to those for some prior art earplugs.

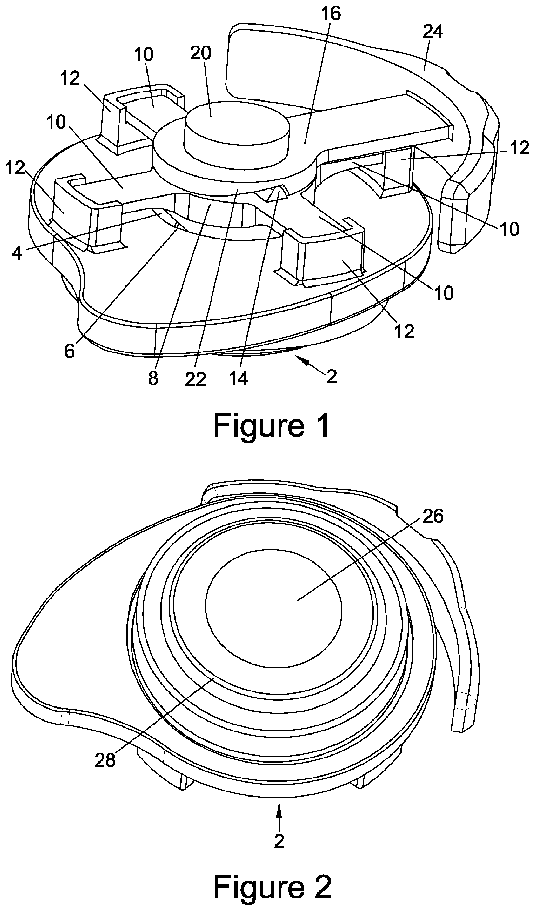

Figure 1 shows an isometric view of a device in accordance with an embodiment of the present invention. The device includes a body 2 comprising a first adjustable acousto-mechanical portion in the form of an adjustable channel 4. The channel 4 is defined by the space between the wall of a cavity 6 and a piston 8 which extends into the cavity 6. The piston 8 is arranged to move axially within the cavity 6 and is mounted to the body 2 by four resilient arms 10 which are received in appropriately shaped arm receiving portions 12 provided on the body 2. The piston 8 further comprises a ridge 14, the purpose of which will become apparent with discussion of later Figures.

The device further comprises an actuation member 16, which forms part of an adjustment arrangement, arranged to move the piston 8 axially within the cavity 6. The actuation member 16 comprises a cylindrical boss 20 for rotatably mounting the actuation member 16. The cylindrical boss 20 is received in an appropriately shaped socket (not shown in the Figures). The actuation member 16 comprises a set of two inclined cam surfaces 22, one of which can be seen in this Figure. The other cam surface 22 is arranged opposite the cam surface 22 seen in this Figure. The cam surfaces 22 interact with the ridge 14 on the piston 8 which acts as a follower to convert rotation of the actuation member 16 into axial movement of the piston 8. This will be described in more detail below. The actuation member 16 further comprises an actuation portion 24 which may be acted upon to drive rotation of the actuation member 16. This actuation portion 24 may, for example, be acted on directly by a user. Accordingly, in this embodiment, the actuation member 16 comprises an integrated user operable member. Of course, as will be appreciated by those skilled in the art, the actuation member 16 may alternatively be driven by an electric motor.

Figure 2 shows a view of the underside of the device seen in Figure 1. Arranged at the base of the body 2 is a second adjustable acousto-mechanical portion in the form of an adjustable membrane 26. The adjustable membrane 26 is bonded to a rim 28 of the body 2.

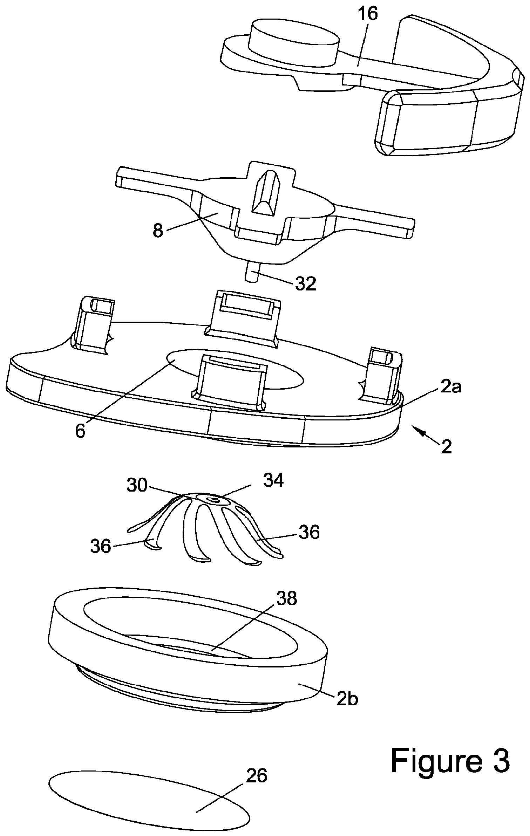

Figure 3 shows an exploded view of the device seen in Figure 1. Starting from the top, there is shown the actuation member 16 and piston 8. In this particular embodiment, the body 2 has a two-part construction comprising a first part 2a and second part 2b although it will be recognised that a one-part construction could be used instead. These two parts are secured together using any appropriate means, e.g. gluing. Also shown in this Figure is a resilient member 30 which acts between the piston 8 and the second part 2b of the body 2. The piston 8 comprises a cylindrical protrusion 32 arranged to be received in a circular hole 34 on the resilient member 30 thereby coupling the piston 8 and the resilient member 30. As will be appreciated by those skilled in the art, when the cylindrical protrusion 32 is received in the circular hole 34 of the resilient member 30, when all of the components of the device are assembled, axial movement of the piston 8 downwards into the cavity 6 will act to compress the resilient member 30.

The resilient member 30 comprises a plurality of resilient legs 36 which extend in a spiral-like manner away from the circular hole 34. When assembled, the resilient legs 36 engage with an internal ledge 38 on the second part 2b of the body 2. The membrane 26 is bonded to the second part 2b of the body 2, as seen in Figure 2.

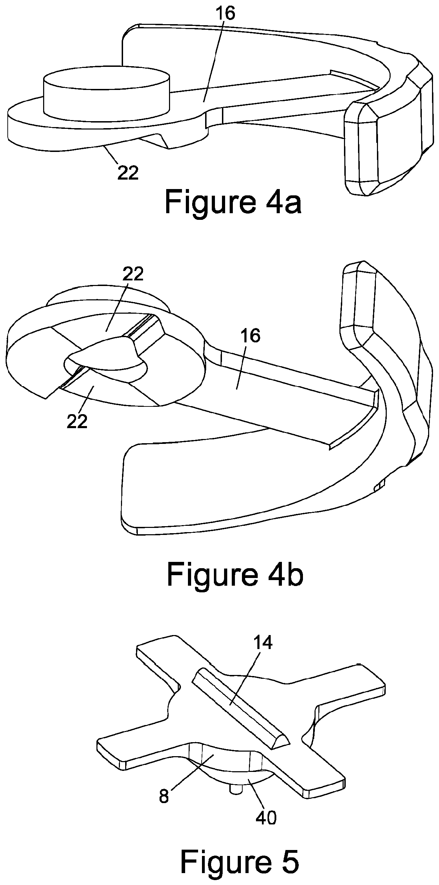

Figures 4a and 4b show a view of the actuation member 16 in isolation from the other components of the device. The inclined cam surfaces 22 can be seen more clearly in these Figures. Fig. 4b shows a view of the underside of the actuation member 16 and shows the presence of both cam surfaces 22 on the underside of the actuation member 16.

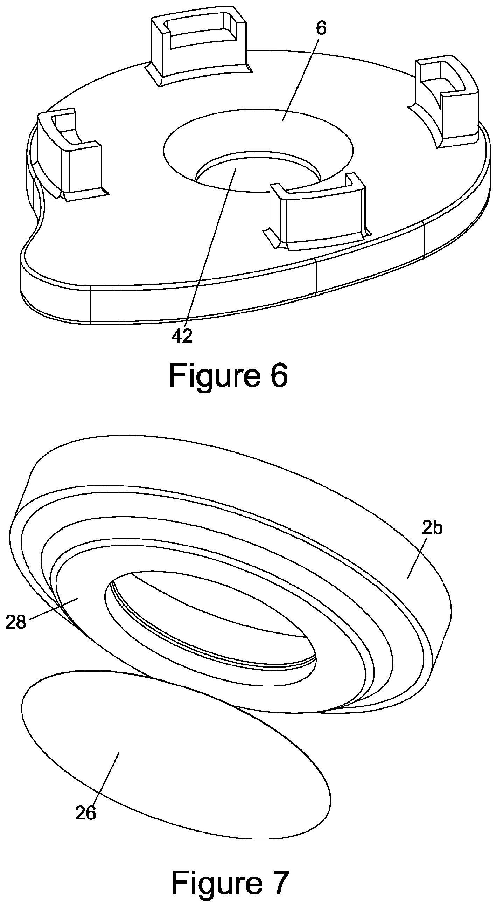

Figure 5 shows an isometric view of the piston 8 in isolation from the other component of the device. The lowermost portion 40 of the piston 8, which extends into the cavity 6 when the device is assembled, is frustoconically shaped and has a corresponding shape to the cavity 6 (seen in Figure 6). As will be appreciated by those skilled in the art, the cavity 6 and lowermost portion 40 of the piston 8 which have a complementary frustoconical shape create an annular channel 4 with a uniform shape and reducing diameter along its length. The ridge 14 extends linearly along the top of the piston and is arranged to interact with the cam surfaces 22 on the actuation member 16 seen in earlier Figures.

Figure 6 shows an isometric view of the upper part 2a of the body 2 in isolation. It can be seen more clearly in this Figure how the cavity 6 has a frustoconical shape. At the base of the cavity 6 is an aperture 42 through which at least part of the piston 8 may extend. This aperture 42 allows at least the cylindrical protrusion 32, on the piston 8, to pass through the cavity 6 and interact with the resilient member 30. It also allows sound to more freely pass out through the cavity 6 into the rest of the device.

Figure 7 shows a view of the underside of the lower part 2b of the body 2 along with the membrane 26. The internal ledge 38 of the lower part 2b of the body 2 seen in Figure 3, also defines an external rim 28 to which the membrane 26 is bonded when the components are assembled. As will be appreciated by those skilled in the art, the external rim 28 is relatively large when compared to the size of the membrane 26 and thus provides a substantial surface area on the lower part 2b of the body 2 on which to bond the membrane 26. This may help to ensure that the membrane 26 is securely bonded to the lower part 2b of the body 2.

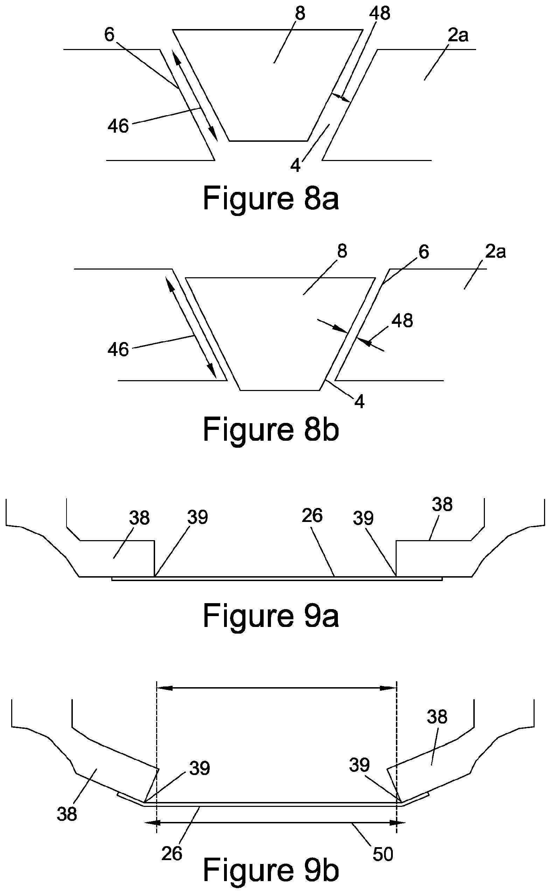

Figures 8a-8b are illustrations demonstrating how the position of the piston 8 in the cavity 6 can be used to adjust the channel 4. When the piston 8 is in the position seen in Figure 8a, the channel 4 has a length shown by arrow 46 and a width shown by arrow 48. As demonstrated by Figure 8b, when the piston 8 is moved axially into the cavity 6, the length of the channel shown by arrow 46 and the width of the channel shown by arrow 48 are both changed. Accordingly, changing the axial position of the piston 8 relative to the cavity 6 will adjust the channel 4. As will be appreciated by those skilled in the art, adjusting the dimensions of the channel 4 will serve to alter the acoustic response of the channel 4 and thus alter the acoustic response of the sound path. More specifically the width, d, of the channel is related to the acoustic loss and the acoustic mass of the channel. Under the electrical circuit analogy for analysis of acoustic systems which will be familiar to those skilled in the art, the acoustic loss is equivalent to a resistance R and has an inverse cube relationship to the channel width as shown below:

$$R = k_1 \frac{1}{d^3} \qquad\qquad \text{(Eq 1).}$$

where $k_1$ is a constant representing parameters assumed to remain constant such as air density and dimensions.

Under the same electrical analogy, the acoustic mass is equivalent to an inductance, L and has an inverse relationship to the channel width, d:

$$L = k_2 \frac{1}{d} \qquad\qquad \text{(Eq 2).}$$

where $k_2$ is a constant representing parameters assumed to remain constant such as air density and dimensions.

The resistance R and the inductance L are both directly proportional to the length of the channel.

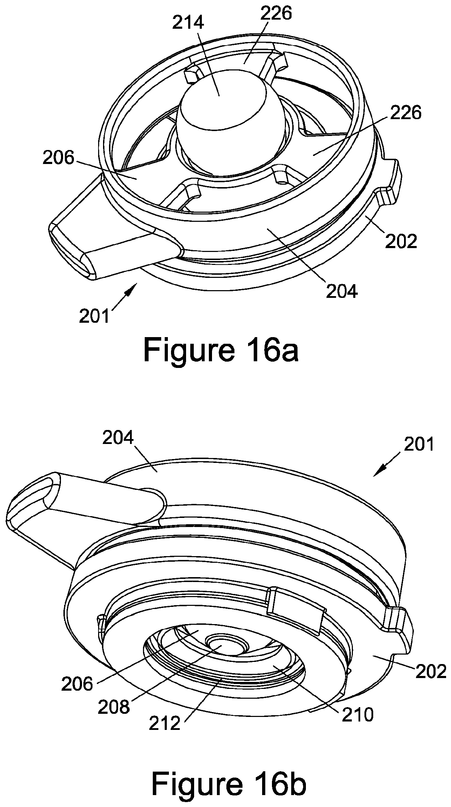

Figures 9a-9b are illustrations demonstrating how the tension of the membrane 26 is adjusted. Figure 9a shows the membrane 26 under a first tension which, for the purposes of illustration, may correspond to the piston 8 being in the position seen in Figure 8a. The membrane 26 extends between the ledge 26 and has a length 50 extending between the corners 39 of the ledge 38. When a force is applied to the ledge 38 it will be caused to bend, as demonstrated by Figure 9b. When the ledge 38 bends, the corners 39 move away from one another thereby stretching the membrane 26. This can be seen as the length 50 between the corners 39 of the ledge 38 is larger in Figure 9b than it is in Figure 9a. Following the aforementioned electrical analogy, when the membrane is tensioned the acoustic capacitance will decrease. As this capacitance is in series with the acoustic capacity of the ear canal, but also the resistance and inductance of the transmission path through the channel, this will cause the attenuation will increase, but will also change the frequency response. In accordance with the invention the changes in acoustic resistance, inductance and capacitance can be tuned so that the change in frequency response matches the natural frequency response of the human ear so that the frequency spectrum perceived by a user is essentially flat as may be seen below with reference to Fig. 16.

The device seen in the earlier Figures may form part of a device which is inserted into the ear canal of a mammalian subject. For example, the device may be embedded within a foam, or other appropriate material, insert which is suitably shaped for insertion into the ear canal of a mammalian subject. The insert may be a standard insert which is suitable for a variety of different ear shapes, or alternatively it may be a custom moulded insert which is specific for a particular user.

Operation of the device will now be described with reference to Figures 1-9. In use, when inserted into the ear of a mammalian subject, the device is arranged such that the membrane 26 is proximal to a user's eardrum and the actuation member 16 projects outwards from the user's ear. This is not essential to the invention and other embodiments are envisaged in which the order of the channel and the membrane is reversed. Accordingly, sound will pass through the channel 4 and subsequently through the membrane 26 into a user's ear canal.

The actuation member 16 may be used to control the position of the piston 8, and simultaneously adjust the tension on the membrane 26 in order to control the acoustic response of the sound path through the device. As the actuation member 16 is held in a fixed axial position, when the actuation member 16 is rotated, the cam surfaces22 acts on the ridge 14 thereby forcing the piston 8 axially downwards into the cavity 6. The piston 8 is prevented from rotating due to the resilient arms 10 being received in the receiving portions 12 on the body 2. The resilient arms 10 also allow the piston 8 to move axially downwards into the cavity 6. As will be appreciated by those skilled in the art, as the piston 8 is moved into the cavity 6, the length of the channel 4 will increase, and its cross section will reduce, as demonstrated earlier in Figures 8a-8b.

Further, as the piston 8 moves into the cavity 6, due to the engagement between the piston 8 and the resilient member 30, the piston will also act to compress the resilient member 30. As the resilient member 30 is compressed it will apply a force to the ledge 38, thereby causing it to deform in a manner which results in the tension on the membrane 26 being increased. This is demonstrated in Figures 9a-9b.

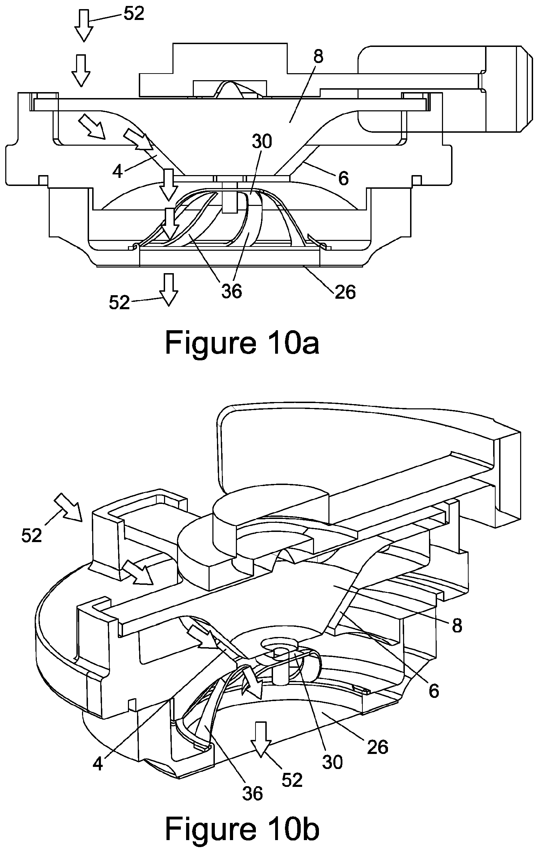

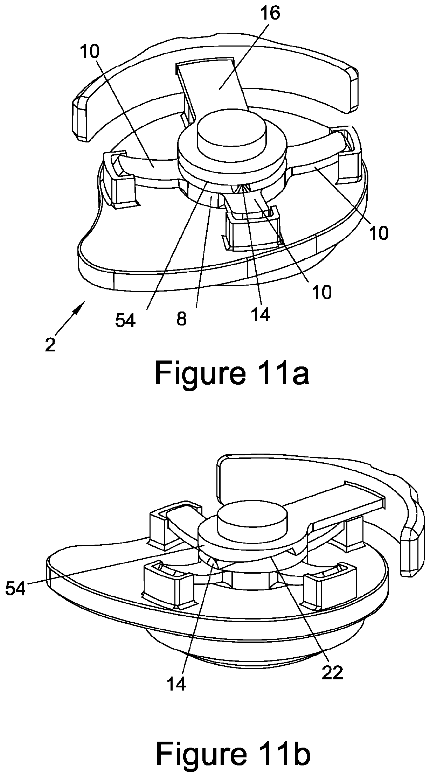

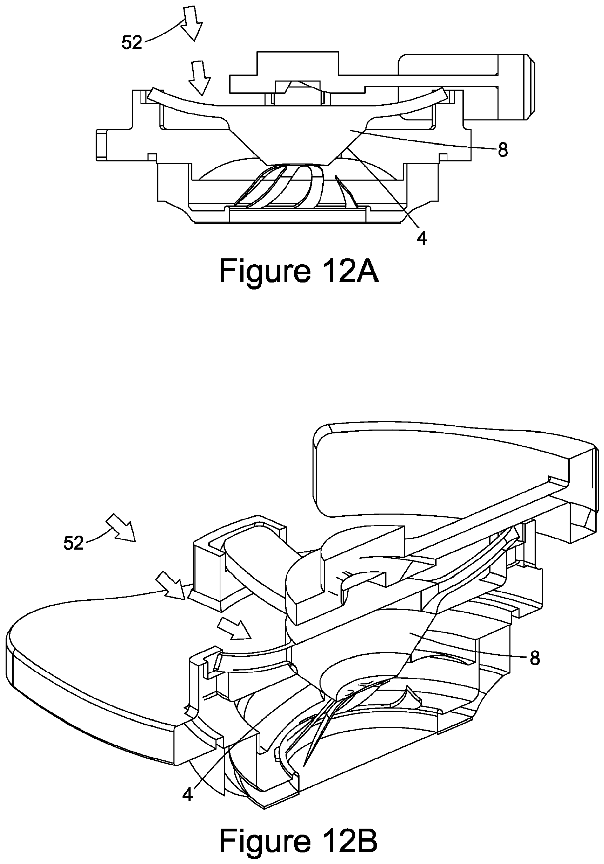

Adjusting the length and cross-sectional area of the channel 4, along with adjustment of the tension of the membrane 26 alters the acoustic response of the sound path through the device. Changing the acoustic response of the sound path will alter the sound heard by a user of the device. As will be appreciated by those skilled in the art, with this embodiment the piston 8 may be driven to any one of a large number of positions between the channel 4 being in a fully 'open' position and the channel being in a fully 'closed' position. Figures 10a and 10b show the device with the piston in the fully open position and Figures 11a, 11b, 12a and 12b show the device with the piston in the fully closed position. This alters the acoustic resistance, inductance and capacitance as previously explained

Figures 10a and 10b show a cross-sectional view through the device with the piston 8 in the position seen in Figure 1, i.e. in a fully open position, with Figure 10a showing the device side-on and Figure 10b showing the device in isometric view. The arrows 52 represent the sound path through the device. As can be seen in each of these Figures, sound enters the device and passes through the channel 4. The sound can freely pass through the gaps between the legs 36 on the resilient member 30. The sound then propagates towards, and passes through, the membrane 26. These Figures also more clearly show how the channel 4 has a frustoconical shell shape due to the frustoconical cavity 6 and the frustoconical piston 8.

Figures 11a and 11b show two different isometric views of the device in a second 'closed' configuration. In this configuration the actuation member 16 has been rotated relative to the body 2 so as to drive the piston 8 downwards by its maximum amount. As the piston 8 is moved downwards, the resilient arms 10 deform to permit this movement. As can be seen most clearly in Figure 11b, the actuation member 16 has been rotated by a sufficient amount that the graduated drive portion 22 has moved past the ridge 14 on the piston 8 and the ridge 14 now rests against a flat portion 54 on the actuation member 8. As will be appreciated by those skilled in the art, once the ridge 14 rests against the flat portion 54 it cannot be driven any further downwards.

Figures 12a and 12b show cross-sectional views through the device in the closed position. The piston 8 has been moved down to a position in which the channel 4 is completely closed. In this position, the sound 52 will no longer be able to pass through the channel 4 and the device may completely, or at least substantially, prevent any sound from passing therethrough.

With reference to earlier Figures, as will be appreciated by those skilled in the art, when it is desired to re-open, at least partially, the channel 4, a user may rotate the actuation member 16 in the opposite direction. The resilient arms 10 will bias the piston 8 axially upwards out of the cavity 6 so as to re-open the channel 4. Of course, due to the coupling between the piston 8 and membrane 26, via the resilient member 30, as the piston 8 moves axially upwards it will gradually release the tension on the membrane 26.

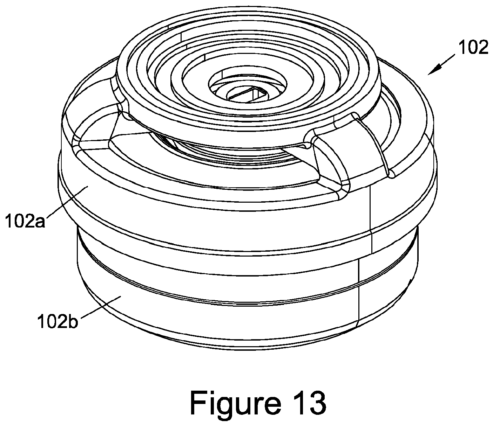

Figure 13 shows an isometric view of a device in accordance with a second embodiment of the present invention. The device comprises a body 102 comprising an upper part 102a and lower part 102b.

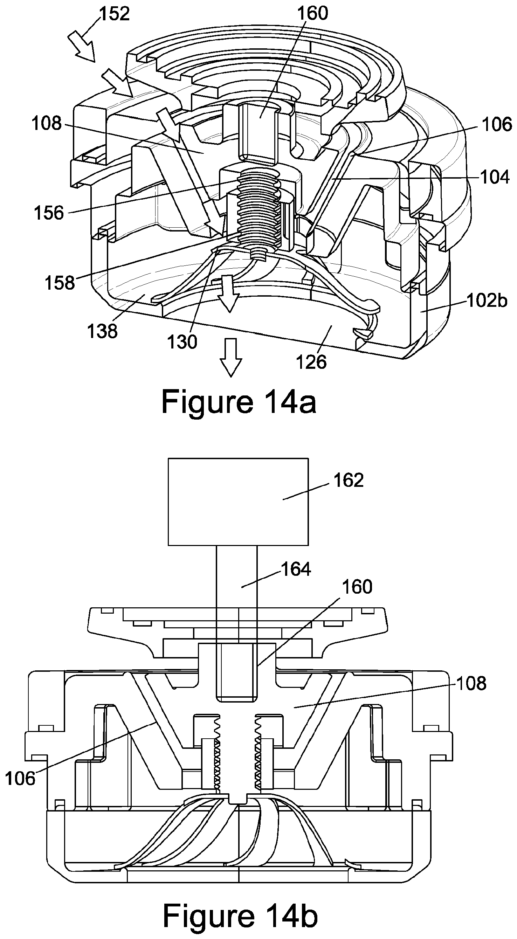

Figure 14a shows a cross-sectional view through the device seen in Figure 13. The device comprises many of the same components as the device seen in Figure 1 including a channel 104 which is defined by the space between a cavity 106 and piston 108. A membrane 126 is bonded to the base of the lower body 102b. Arranged between the membrane 126 and piston 108 is a resilient member 130. The resilient member 130 is identical to the resilient member 30 described previously. Similarly to the earlier embodiment, the resilient member 130 acts on an internal ledge 138.

The device according to this embodiment differs from the first embodiment, seen in Figure 1, in that the piston 108 is rotatably mounted in the body 102. The piston 108 comprises an external thread 156 which engages with a corresponding threaded portion 158 on the body 102. Accordingly, as will be appreciated by those skilled in the art, when the piston 108 is rotated, it is caused to advance into, or be drawn out of, the cavity 106. The direction of rotation of the piston 108 will dictate whether it is advanced into, or drawn out of, the cavity 106. The top of the piston 108 is provided with a rectangular slot 160. A drive shaft, for example connected to a motor, (not shown in this Figure), may be inserted into the slot 160 in order to drive rotation of the piston 108. In the view shown in Figure 14a, the device is in an 'open' configuration in which the sound path 152 is open to allow sound to pass through the device.

Figure 14b shows the device according to the second embodiment in cross-sectional view when viewed side-on. A motor 162 and drive shaft 164, which is inserted into the key hole 160, may be used to drive rotation of the piston 108 and thus advance it into, and draw it out of, the cavity 106.

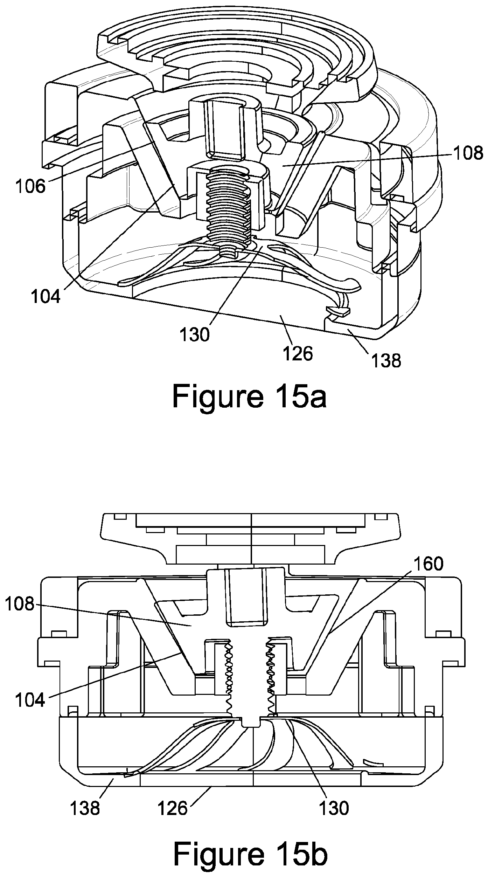

Figures 15a and 15b show cross sectional view through the device in a 'closed' configuration, with the motor 162 and drive shaft omitted. In this configuration the piston 108 has been advanced into the cavity 106 such that the channel 104 is now closed. As a result, sound can no longer pass through the channel 104 and thus the device will significantly attenuate any incident sound. Whilst not visible in these Figures, in this configuration, the resilient member 130 will be pressed against the internal ledge 138 and thereby cause an increase in the tension on the membrane 126.

Of course, as will be appreciated by those skilled in the art, the piston 108 may be moved to any intermediate position between the open and closed positions seen in Figures 14a-14b and 15a-15b, in order to achieve a desired acoustic response of the sound path.

Figures 16a shows an isometric view of part of a hearing protection device in accordance with a third embodiment of the present invention. As can be seen in Figure 16a, the device 201 includes a body 202 upon which a user operable member in the form of a handle member 204 is located.

The device 201 further comprises a resiliently compressible member 214 which is located upon the handle member 204. The resiliently compressible member 214 is arranged to apply a force to the various components of the device 201 to maintain the handle member 204 and the body 202 in position with respect to each other e.g. when the handle member 204 is rotated. The resiliently compressible member 214 can be compressed by varying degrees.

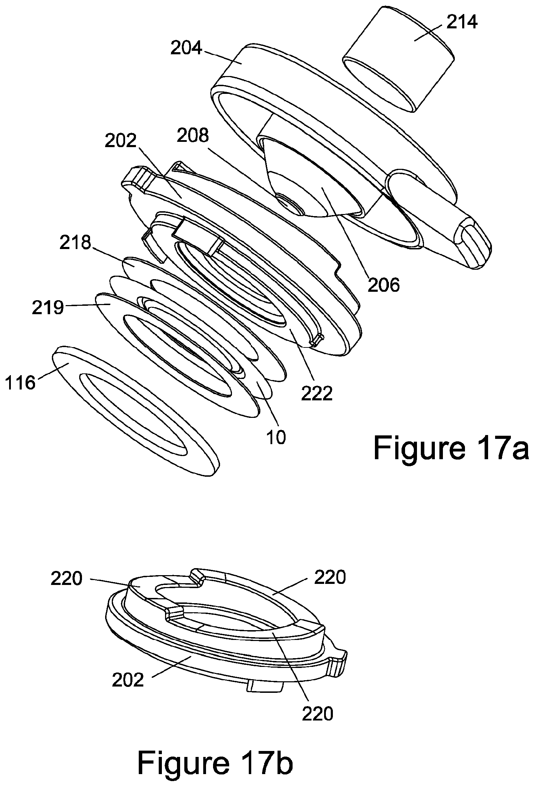

Figure 16b shows an isometric view of the underside of the device shown in Figure 16a. Arranged towards the base of the device 201 is a circular membrane 210. Whilst the membrane shown in Figure 16b is transparent, this is not essential and the membrane could be opaque. The membrane is attached to the body 202 of the device, in particular bonded to a circular rim 222 (as can be seen in Figure 17a) formed within the body 202 of the device 201.

As can be seen from Figure 16b, the handle member 204 is integrally formed with a piston 206 which comprises a compressible portion 208 at its distal end in the form of a disc of compressible material such as a closed cell foam or thermoplastic elastomer. As will be explained later, the compressible portion 208 is arranged to contact the membrane 210 to increase the tension of the membrane 210. The piston 206 is attached to the handle member 204 by means of three spokes 226 which extended from the circumference of the handle member 204 to the piston 206 so that movement of the handle member 204 results in the movement (e.g. adjustments) of the piston 206. The movement of the piston 206 changes the degree of compression of the compressible member 214.

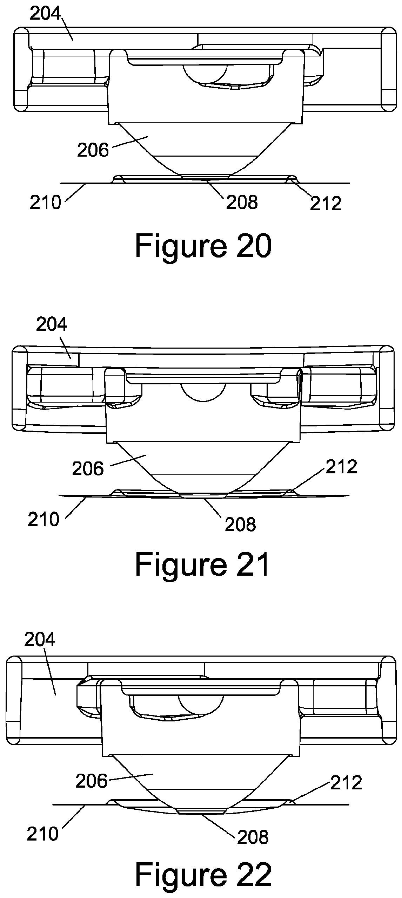

The handle member 204 can be rotated with respect to the body 202 of the device 1. In particular, the handle member 204 may be moved by a user to a variety of different rotation positions with respect to the body 202 of the device. When the handle member 204 is rotated, the piston 206 also rotates. The conversion of rotational movements of the handle member 204 to axial (linear) movement of the piston 206 will be discussed in more detail in relation to Figures 20-25. As will also be discussed in further detail in relation to Figures 20-25, different rotational positions of the handle member 204 correspond to different attenuations provided by the device 201.

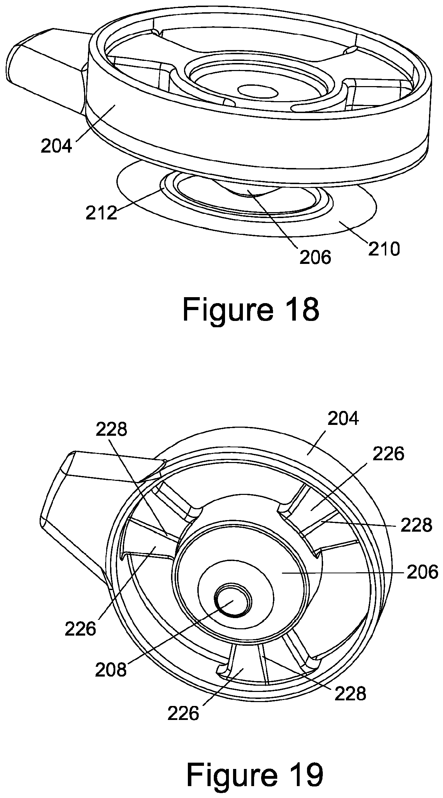

The membrane 210 further comprises a corrugation 212. The corrugation 212 can be seen more clearly in Figure 18 which shows an isometric view of the isolated handle member 204 and membrane 210 of the device shown in Figure 16a. The corrugation 212 is a circular ridge centred on the geometric centre of the membrane 210 which extends above the plane of the membrane 210. This arrangement of the corrugation 212 means that the compressible portion 208 does not come into contact with the corrugation 212 (e.g. the compressible portion 208 contacts the membrane 210 in the geometric centre of the membrane 210). The cross-section of the ridge resembles a bell curve, which can be seen more clearly in the cross-sectional views seen in Figure 20-22.

Figure 17a shows an exploded view of the device seen in Figure 1. Starting from the top, Figure 17a shows the resiliently compressible member 214 and the handle member 204. The handle member 204 is essentially circular with a protruding grip portion. As previously discussed, the handle member 204 is integrally formed with a piston 206. The piston 206 extends below the handle member 204 and is concentric with it. Figure 17a also shows the tensioned membrane 210 which comprises the aforementioned corrugation 212.

In Figure 17a, the rim 222 which forms part of the body 202 of the device 1 can be seen. The membrane 210 is secured in position on the rim 222 by a stabilising ring 216 and two adhesive tape rings 218, 219. The stabilising ring 216 and tapes 218, 219 have the same shape as the rim 222. When the device is constructed, the first tape 218 is positioned between the rim 222 and the membrane 210. The second tape 219 is positioned between the membrane 210 and the stabilising ring 216. The stabilising ring 216 holds the membrane 210 in position during formation of the corrugation 212 in the production process.

In the embodiment of the device shown in Figure 17b, the body 202 includes three inclined cam surfaces 202, located on the uppermost surface of the body 202. Figure 19 shows an isometric view of the underside of the handle member 204 and piston 206 in isolation. In this isolated view, the underside of each of the spokes 226 of the handle member 204 can be seen to comprise a ridge 228 on its underside. When the device is assembled (e.g. as seen in Figures 16a and 16b), each of the ridges 228 contacts a corresponding inclined cam surface 220 of the body 202 of the device 201.

Operation of the device will now be described with reference to Figures 20 to 25. Figures 8a-8b are illustrations demonstrating how the position of the piston 8 in the cavity 6 can be used to adjust the channel 4 which are equally applicable to the third embodiment of the present invention shown in Figures 16-25.

Figures 20-22 are illustrations demonstrating how the position of the piston 206 can be used to adjust the tension of the membrane 210. For clarity, these illustrations show a cross-section through the handle member 204, the piston 206 and the membrane 210 in isolation from the other components of the device 201.

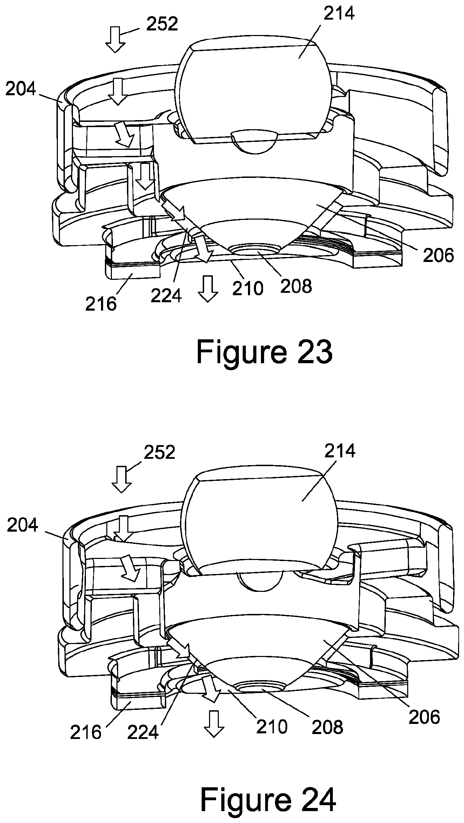

Figures 23-25 are illustrations demonstrating how the position of the piston 206 can also be used to adjust the dimensions of a channel 224. In the embodiments shown in Figures 20-25, the piston 206 simultaneously adjusts the dimensions of the channel 224 and the tension of the membrane 210.

When the handle member is in the first position shown in Figures 20 and 23, each ridge 228 contacts the corresponding inclined cam surface 220 at its highest point.

As a result of this, the piston 206 is not in contact with the membrane 210. In this position, the piston 206 applies zero force to the membrane 210. The tension in the membrane 210 is therefore the base tension (i.e. the minimum tension in the membrane). This base tension is provided by the attachment of the membrane 210 to the rim 222 of the body 202. The compressible portion 208 is in a compressed state, which biases the movement of the piston 206 axially downwards towards the membrane 10.

As shown in Figure 23, when the handle member 204 is in the first position, a channel 224 is formed between the wall 230 of the cavity formed by the body 202 of the device 201 and the piston 206. The arrows illustrate how sound propagates through the device along a sound path 252 which includes the channel 224.

The handle member 204 may then be rotated by the user to a second position, as shown in Figures 21 and 24. In the second position of the handle member 204, each ridge 228 contacts the corresponding inclined cam surface 220 at the middle point of the inclined cam surface 220. When the handle member is rotated from the first position to the second position, each ridge moves down the corresponding inclined cam surface 220. The whole of the handle member 204 moves axially downwards towards the body 202 of the device 201 and therefore the piston 206 is moved axially downwards towards the membrane 210. Together, the inclined cam surfaces 220 and ridges 228 on the spokes 226 convert the rotational movement of the handle member 204 to axial movement of the piston 206.

In the second position shown in Figure 21, the piston 206 is in a position in which the piston 206 just contacts the surface of the membrane 210. In the absence of a corrugation, when the piston 206 contacts the membrane 210, the primary vibration mode of the membrane 210 is disabled and the centre of the membrane 210 can be considered to be held stationary. Therefore, only higher order harmonic vibration modes of the membrane 210 are enabled, resulting in an abrupt increase in the attenuation. Including the corrugation 212 in the membrane reduces this effect. Therefore, the level of attenuation is more smoothly increased when the piston just contacts such a membrane 210 which includes a corrugation 212.

As the piston 206 comprises a compressible portion 208, some of the force which would have otherwise been exerted on the membrane 210 (e.g. compared with a rigid piston with no compressible portion) acts to compress the compressible portion 208. This results in a smaller force being exerted on the membrane 210 by the piston 206 and therefore a smaller increase in tension of the membrane 210. The level of attenuation is therefore increased more gradually when the piston 206 is moved to just contact the surface of the membrane 210 (compared with a rigid member).

In the second position as shown in Figure 24, the piston 206 is positioned so as to decrease the dimensions (e.g. the width and/or the length) of the channel 224 compared with the first position. As previously described, decreasing the width of the channel 224 increases the effective acoustic mass and the acoustic loss of the channel 224. Decreasing the length of the channel 224 decreases the effective acoustic mass and the acoustic loss of the channel 224. This, together with the altered tension of the membrane 210, changes the overall response of the device 201.

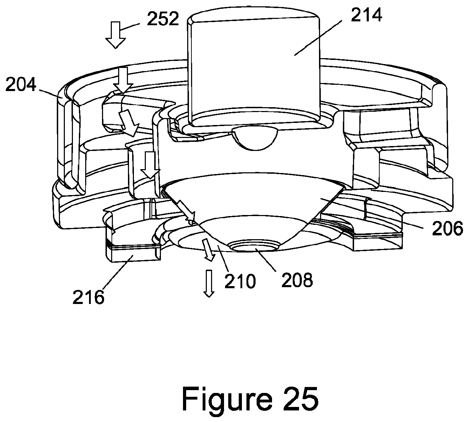

The handle member 204 may be further rotated by the user to a third position, as shown in Figure 22 and 25. In the third position of the handle member 204, each ridge 228 contacts the corresponding inclined cam surface 220 at the lowest point. This corresponds to limit of rotational movement of the handle member 204 (in this direction) and therefore the limit of axial movement of the piston (towards the membrane).

In the third position shown in Figure 207, the piston is in contact with the surface of the membrane, and exerts a larger (e.g. a maximum) force on the membrane (e.g. than was exerted when the handle member 204 was in a second position and the piston 206 just contacted the surface of the membrane 210). The force exerted by the piston 206 also causes a more pronounced deformation of the rest of the membrane 210. As can be seen in Figure 22, in this position the membrane 210 is deformed from essentially planar to concave or frusto-conical as a result of the force exerted on the membrane 210 by the piston 206. In this configuration, the tension of the membrane 210 is increased further above the base tension (e.g. the tension is increased to its maximum). This results in the attenuation provided by the device 201 being greater (i.e. than the attenuation provided by the device 201 when the handle member is in the first or second positions shown in Figures 20 and 21 respectively).

In the third position shown in Figure 25, the piston 206 contacts the walls of the cavity in body 202 of the device 201. Therefore, there is a minimal channel width (e.g. no channel) between the walls of the cavity of the body and the piston 206. In this arrangement, the minimal channel width together with the high tension of the membrane, both results in the device providing its maximum attenuation. The compressible member 214 is in its least compressed state.

In embodiments in which the attenuation provided by the device is at a minimum, when the handle member is in the first position, the channel 224 may be described as 'open' and the device 201 may be described as being in an 'open' configuration. In embodiments in which the attenuation provided by the device is at a maximum when the handle member is in the third position, the channel 224 may be described as 'closed' and the device 201 may be described as being in a 'closed' configuration.

Of course, as will be appreciated by those skilled in the art, the handle member 204 may be moved to any intermediate position between the first, second and third positions seen in Figures 20-22 and 23-25, in order to achieve a desired acoustic response of the sound path 252.

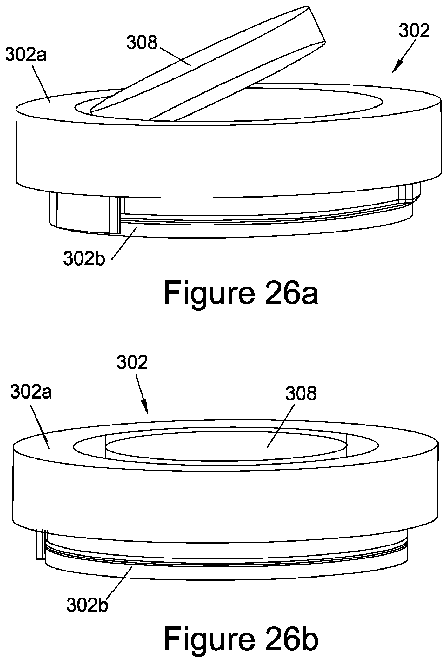

Figure 26a-26b show cross-sectional views of a device in accordance with a fourth embodiment of the present invention, comprising a body 302 formed from an upper part 302a and a lower part 302b. The device also comprises a membrane (not shown) and a lid 308, wherein the lid 306 is arranged to provide additional attenuation. For example, the lid 306 may be used to minimise sound entering the device when a user is in an environment with a high sound pressure level. In Figure 26a the lid is shown in an open configuration, in which the sound path (not shown) is open in order to allow sound to pass into the device. In Figure 26b the lid is shown in a closed configuration, in which the sound path (not shown) is open in order to allow sound to pass through the device. A hinge may be used to allow the lid to be moved, either manually or automatically, from the open to the closed position (and vice versa).

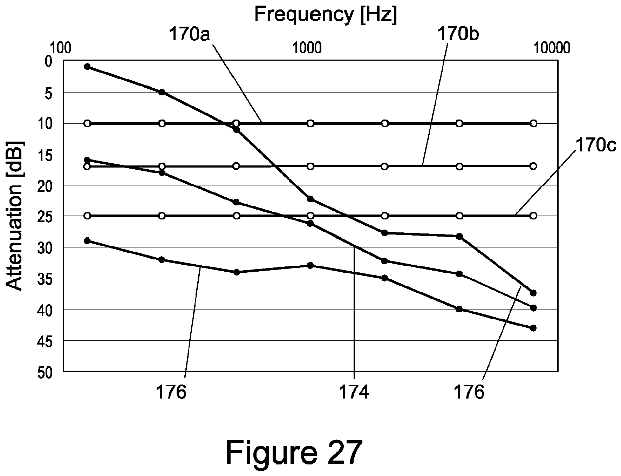

Fig. 27 shows a graph comparing the frequency response of an embodiment of the invention to those of three prior art passive earplugs. The graph shows the relationship between the frequency on the horizontal axis and attenuation on the vertical axes. Both axes have logarithmic scales. The attenuation shown is that relative to the natural, unimpeded frequency response of the human aural system. It therefore takes into account the well-known typical variation of sensitivity that humans have dependent on frequency.

The uppermost substantially horizontal plot 170a corresponds to an embodiment of the invention similar to that described above with the piston almost fully open. As may be seen the attenuation provided is essential constant across the frequency spectrum. This means that a user will experience sounds. Similarly plot 170b shows the situation when the piston is half-closed. Here a greater attenuation is provided but it is still substantially constant with frequency. The lowermost plot shows the response when the piston is almost closed. Here the attenuation is at a maximum (approximately 25 dB) but remains substantially constant with frequency.

By contrast plots 172, 174 and 176 show respective frequency responses for a typical passive earplug which has been inserted into a user's ear canal to differing degrees. The uppermost plot 172 represents the earplug being inserted by the least amount (significantly less than it is intended to be). This means that at low frequencies there is almost no attenuation at all (which could be dangerous). The other plots 174, 176 show that earplug being inserted more fully and therefore being more effective. However, as may be seen, there is a substantial increase in attenuation with frequency in all three cases meaning that higher frequency sounds are disproportionately filtered out compared to lower frequencies. The result of this is that the user experiences sounds as muffled which may lead to problems with intelligibility of speech when listening to co-workers for example. This might encourage the user to remove the earplug or not to insert it properly, thereby making it less effective than it might be.

**Claims**

1.  A device for insertion into an ear canal of a mammalian subject, comprising:

    a body (2), having at least one sound path (52) extending therethrough;
    a first adjustable acousto-mechanical portion comprising an adjustable channel (4) forming at least part of the sound path (52) **characterised by**;
    a second adjustable acousto-mechanical portion, arranged acoustically in series with the first adjustable acousto-mechanical portion, comprising an adjustable membrane (26); and
    an adjustment arrangement for simultaneously adjusting the first and second adjustable acousto-mechanical portions to alter an acoustic response of the at least one sound path (52).

2.  The device as claimed in claim 1, wherein the adjustment arrangement comprises a common actuator arranged to adjust both the first and second acousto-mechanical portions simultaneously, optionally wherein the actuator comprises a user operable member (24) arranged to operate at least part of the adjustment arrangement.

3.  The device as claimed in any preceding claim, wherein the adjustment arrangement is configured to adjust a length and/or a width of the adjustable channel (4).

4.  The device as claimed in any preceding claim, wherein the channel (4) is defined by a space between a wall of a cavity (6) within the body and a piston (8) arranged in the cavity (6), wherein adjustment of the channel (4) is achieved by moving the piston (8) relative to the cavity (6), optionally wherein the cavity (6) and the piston (8) each have a frusto-conical shape such that the adjustable channel (4) has the form of a frusto-conical shell.

5.  The device as claimed in claim 4, wherein the piston (8) is arranged to move axially within the cavity (6) and the device comprises at least one resilient member (30) arranged to bias the piston (8) out of the cavity (6), wherein the adjustable arrangement comprises an actuation member (16) arranged to drive the piston (8) against the resilient bias axially into the cavity (6), optionally wherein the actuation member (16) is arranged to rotate relative to the piston (8), and the device further comprises an arrangement for converting rotational movement of the actuation member (16) into axial movement of the piston (8).

6.  The device as claimed in any claim 4 or 5, wherein the piston (8) is arranged such that movement of the piston (8) adjusts a tension of the membrane (26).

7.  The device as claimed in claim 6, wherein the piston (8) is coupled to a resilient member (30) arranged to act on the membrane (26), or a part of the body (2b) to which the membrane (26) is attached.

8.  The device as claimed in claim 6, wherein the piston (8) is arranged to contact the membrane (26), optionally wherein the piston (8) comprises a compressible portion (208).

9.  The device as claimed in claim 8, wherein the compressible portion (208) of the piston contacts the surface of the membrane (26).

10. The device as claimed in claim 8 or 9, wherein the piston (8) has a position wherein the piston (8) is not in contact with the membrane (26) and does not alter the tension of the membrane (26) from a base value.

11. The device as claimed in any one of claims 8 to 10, wherein the piston (8) has a plurality of positions wherein the piston (8) is in contact with the membrane (26) corresponding to a plurality of magnitudes of force applied to the membrane (26) and a plurality of tensions in the membrane (26).

12. The device as claimed in any preceding claim, wherein the channel (4) comprises an adjustable barrier member.

13. The device as claimed in any preceding claim, wherein the membrane (26) is a separate component attached to the body (2) and the body (2) defines a circumferential rim (28) to which the membrane (26) is attached.

14. The device as claimed in any preceding claim, wherein the membrane (26) comprises at least one corrugation (212), optionally wherein the corrugation (212 is circular and centred on the geometric centre of the membrane (26), optionally wherein the membrane (26) comprises a plurality of corrugations (212).

15. The device as claimed in any preceding claim further comprising a controller arranged to control the adjustment arrangement so as to alter the acoustic response of the at least one sound path (52) and/or a user input arranged to enable the user to control operation of the device.


**Patentansprüche**

1. Vorrichtung zum Einsatz in einen Gehörgang eines Säugetiersubjekts, umfassend:

    einen Körper (2), der mindestens einen Schallweg (52) aufweist, der sich dadurch erstreckt;
    einen ersten anpassbaren akustisch-mechanischen Abschnitt, der einen anpassbaren Kanal (4) umfasst, der mindestens einen Teil des Schallwegs (52) bildet, **gekennzeichnet durch**;
    einen zweiten anpassbaren akustisch-mechanischen Abschnitt, der akustisch in Serie mit dem ersten anpassbaren akustisch-mechanischen Abschnitt angeordnet ist und eine anpassbare Membrane (26) umfasst; und
    eine Anpassungsanordnung zum gleichzeitigen Anpassen des ersten und des zweiten anpassbaren akustisch-mechanischen Abschnitts, um eine akustische Reaktion des mindestens einen Schallwegs (52) zu verändern.

2. Vorrichtung nach Anspruch 1, wobei die Anpassungsanordnung ein gemeinsames Stellglied umfasst, das angeordnet ist, um sowohl den ersten als auch den zweiten akustisch-mechanischen Abschnitt gleichzeitig anzupassen, wobei das Stellglied optional ein benutzerbedientes Element (24) umfasst, das angeordnet ist, um mindestens einen Teil der Anpassungsanordnung zu bedienen.

3. Vorrichtung nach einem vorstehenden Anspruch, wobei die Anpassungsanordnung konfiguriert ist, um eine Länge und/oder eine Breite des anpassbaren Kanals (4) anzupassen.

4. Vorrichtung nach einem vorstehenden Anspruch, wobei der Kanal (4) durch einen Raum zwischen einer Wand eines Hohlraums (6) innerhalb des Körpers und einem Kolben (8), der in dem Hohlraum (6) angeordnet ist, definiert ist, wobei Anpassung des Kanals (4) durch Bewegen des Kolbens (8) im Verhältnis zu dem Hohlraum (6) erreicht wird, wobei der Hohlraum (6) und der Kolben (8) optional jeweils eine kegelstumpfförmige Form aufweisen, so dass der anpassbare Kanal (4) die Form einer kegelstumpfförmige Schale aufweist.

5. Vorrichtung nach Anspruch 4, wobei der Kolben (8) angeordnet ist, um sich axial innerhalb des Hohlraums (6) zu bewegen, und die Vorrichtung mindestens ein elastisches Element (30) umfasst, das angeordnet ist, um den Kolben (8) aus dem Hohlraums (6) vorzuspannen, wobei die anpassbare Anordnung ein Betätigungselement (16) umfasst, das angeordnet ist, um den Kolben (8) gegen die elastische Vorspannung axial in den Hohlraum (6) anzutreiben, wobei das Betätigungselement (16) optional angeordnet ist, um im Verhältnis zu dem Kolben (8) zu drehen, und die Vorrichtung weiter eine Anordnung zum Umwandeln von Drehbewegung des Betätigungselements (16) in axiale Bewegung des Kolbens (8) umfasst.

6. Vorrichtung nach einem der Ansprüche 4 oder 5, wobei der Kolben (8) so angeordnet ist, dass Bewegung des Kolbens (8) eine Spannung der Membrane (26) anpasst.

7. Vorrichtung nach Anspruch 6, wobei der Kolben (8) mit einem elastischen Element (30) gekoppelt ist, das angeordnet ist, um auf die Membrane (26) oder einen Teil des Körpers (2b), an dem die Membrane (26) befestigt ist, einzuwirken.

8. Vorrichtung nach Anspruch 6, wobei der Kolben (8) angeordnet ist, um die Membrane (26) zu berühren, wobei der Kolben (8) optional einen komprimierbaren Abschnitt (208) umfasst.

**EP 3 941 400 B1**

9. Vorrichtung nach Anspruch 8, wobei der komprimierbare Abschnitt (208) des Kolbens die Oberfläche der Membrane (26) berührt.

10. Vorrichtung nach Anspruch 8 oder 9, wobei der Kolben (8) eine Position aufweist, in der der Kolben (8) nicht in Berührung mit der Membrane (26) ist und nicht die Spannung der Membrane (26) von einem Grundwert verändert.

11. Vorrichtung nach einem der Ansprüche 8 bis 10, wobei der Kolben (8) eine Vielzahl von Positionen aufweist, in denen der Kolben (8) in Berührung mit der Membrane (26) ist, die einer Vielzahl von Magnituden von Kraft, die auf die Membrane (26) angewendet wird, und einer Vielzahl von Spannungen in der Membrane (26) entsprechen.

12. Vorrichtung nach einem vorstehenden Anspruch, wobei der Kanal (4) ein anpassbares Barriereelement umfasst.

13. Vorrichtung nach einem vorstehenden Anspruch, wobei die Membrane (26) eine getrennte Komponente ist, die an dem Körper (2) befestigt ist, und der Körper (2) einen Umfangskranz (28) definiert, an dem die Membrane (26) befestigt ist.

14. Vorrichtung nach einem vorstehenden Anspruch, wobei die Membrane (26) mindestens eine Wellung (212) umfasst, wobei die Wellung (212 optional kreisförmig ist und in der geometrischen Mitte der Membrane (26) zentriert ist, wobei die Membrane (26) optional eine Vielzahl von Wellungen (212) umfasst.

15. Vorrichtung nach einem vorstehenden Anspruch, weiter umfassend eine Steuerung, die angeordnet ist, um die Anpassungsanordnung so zu steuern, dass die akustische Reaktion des mindestens einen Schallwegs (52) verändert wird, und/oder eine Benutzereingabe, die angeordnet ist, um es dem Benutzer zu ermöglichen, den Betrieb der Vorrichtung zu bedienen.

**Revendications**

1. Dispositif destiné à être inséré dans un conduit auditif d'un sujet mammifère, comprenant :

    un corps (2), présentant au moins une trajectoire acoustique (52) s'étendant à travers celui-ci ;
    une première portion acousto-mécanique réglable comprenant un conduit réglable (4) formant au moins une partie de la trajectoire acoustique (52), **caractérisé par** ;
    une seconde portion acousto-mécanique réglable, agencée acoustiquement en série avec la première portion acousto-mécanique réglable, comprenant une membrane réglable (26) ; et
    un système de réglage destiné à régler simultanément les première et seconde portions acousto-mécaniques réglables pour altérer une réponse acoustique de la au moins une trajectoire acoustique (52).

2. Dispositif selon la revendication 1, dans lequel le système de réglage comprend un actionneur commun agencé pour régler à la fois les première et seconde portions acousto-mécaniques réglables simultanément, éventuellement dans lequel l'actionneur comprend un élément manipulable par l'utilisateur (24) agencé pour commander au moins une partie du système de réglage.

3. Dispositif selon une quelconque revendication précédente, dans lequel le système de réglage est configuré pour régler une longueur et/ou une largeur du conduit réglable (4).

4. Dispositif selon une quelconque revendication précédente, dans lequel le conduit (4) est défini par un espace entre une paroi d'une cavité (6) à l'intérieur du corps et un piston (8) agencé dans la cavité (6), dans lequel le réglage du conduit (4) est réalisé par déplacement du piston (8) par rapport à la cavité (6), éventuellement dans lequel la cavité (6) et le piston (8) présentent chacun une forme tronconique de sorte que le conduit réglable (4) présente la forme d'une coque tronconique.

5. Dispositif selon la revendication 4, dans lequel le piston (8) est agencé pour se déplacer axialement à l'intérieur de la cavité (6) et le dispositif comprend au moins un élément élastique (30) agencé pour solliciter le piston (8) hors de la cavité (6), dans lequel le système réglable comprend un élément d'actionnement (16) agencé pour entraîner le piston (8) contre la sollicitation élastique axialement dans la cavité (6), éventuellement dans lequel l'élément d'actionnement (16) est agencé pour tourner par rapport au piston (8), et le dispositif comprend en outre un système destiné à convertir un mouvement de rotation de l'élément d'actionnement (16) en un mouvement axial du piston (8).

**6.** Dispositif selon l'une quelconque de la revendication 4 ou 5, dans lequel le piston (8) est agencé de sorte qu'un mouvement du piston (8) règle une tension de la membrane (26).

**7.** Dispositif selon la revendication 6, dans lequel le piston (8) est couplé à l'élément élastique (30) agencé pour agir sur la membrane (26), ou une partie du corps (2b) à laquelle la membrane (26) est fixée.

**8.** Dispositif selon la revendication 6, dans lequel le piston (8) est agencé pour être au contact de la membrane (26), éventuellement dans lequel le piston (8) comprend une portion compressible (208).

**9.** Dispositif selon la revendication 8, dans lequel la portion compressible (208) du piston est en contact avec la surface de la membrane (26).

**10.** Dispositif selon la revendication 8 ou 9, dans lequel le piston (8) présente une position dans laquelle le piston (8) n'est pas en contact avec la membrane (26) et n'altère pas la tension de la membrane (26) à partir d'une valeur de base.

**11.** Dispositif selon l'une quelconque des revendications 8 à 10, dans lequel le piston (8) présente une pluralité de positions dans lesquelles le piston (8) est en contact avec la membrane (26) correspondant à une pluralité d'amplitudes de force appliquée à la membrane (26) et à une pluralité de tensions dans la membrane (26).

**12.** Dispositif selon une quelconque revendication précédente, dans lequel le conduit (4) comprend un élément barrière réglable.

**13.** Dispositif selon une quelconque revendication précédente, dans lequel la membrane (26) est un composant séparé fixé au corps (2) et le corps (2) définit un rebord circonférentiel (28) auquel la membrane (26) est fixée.

**14.** Dispositif selon une quelconque revendication précédente, dans lequel la membrane (26) comprend au moins une ondulation (212), éventuellement dans lequel l'ondulation (212) est circulaire et centrée sur le centre géométrique de la membrane (26), éventuellement dans lequel la membrane (26) comprend une pluralité d'ondulations (212).

**15.** Dispositif selon une quelconque revendication précédente comprenant en outre un dispositif de commande agencé pour commander le système de réglage de manière à altérer la réponse acoustique de la au moins une trajectoire acoustique (52) et/ou une entrée utilisateur agencée pour permettre à l'utilisateur de commander le fonctionnement du dispositif.

Figure 1

Figure 2

Figure 3

16

22

## Figure 4a

22

22

22

16

## Figure 4b

14

8

40

## Figure 5

Figure 6

Figure 7

Figure 8a

Figure 8b

Figure 9a

Figure 9b

Figure 10a

Figure 10b

Figure 11a

Figure 11b

Figure 12A

Figure 12B

Figure 13

Figure 14a

Figure 14b

Figure 15a

Figure 15b

Figure 16a

Figure 16b

Figure 17a

Figure 17b

204

212

206

210

## Figure 18

228

204

226

228

226

206

208

226

228

## Figure 19

204

206

210    208    212

## Figure 20

204

206    212

210    208

## Figure 21

204

206    212

210    208

## Figure 22

Figure 23

Figure 24

Figure 25

302a

308

302

302b

# Figure 26a

302a

302

308

302b

# Figure 26b

Figure 27

**EP 3 941 400 B1**

## REFERENCES CITED IN THE DESCRIPTION

### Patent documents cited in the description

- US 7478702 B